# EUROPEAN PATENT APPLICATION

(11) **EP 2 813 850 A1**
(43) Date of publication of application: **17.12.2014**
(21) Application number: 13305778.6
(22) Date of filing: 10.06.2013
(51) Int. Cl.: G01N 33/68

(54) **Methods for predicting rheumatoid arthritis treatment response**

(71) Applicant: INSERM (Institut National de la Santé et de la Recherche Médicale), 75013 Paris (FR)
(72) Inventor: Vittecoq, Olivier, 27310 Saint Ouen de Thouberville (FR); Laguerre, Thierry, 76000 Rouen (FR); Cosette, Pascal, 76130 Mont Saint Aigan (FR); Boyer, Olivier, 76000 Rouen (FR); Le Loët, Xavier, 76000 Rouen (FR); Hardouin, Julie, 76410 Tourville la Rivière (FR); Obry, Antoine, 76130 Mont Saint Aigan (FR)
(74) Representative: Paris, Fabienne

(57) **Abstract**

The present invention relates to methods for predicting rheumatoid arthritis treatment response.

## Description

### FIELD OF THE INVENTION:

The present invention relates to methods for predicting rheumatoid arthritis treatment response.

### BACKGROUND OF THE INVENTION:

Rheumatoid arthritis (RA) is a chronic, auto-immune and inflammatory polyarthritis which induces joint damage and disability. Thanks to the better understanding of RA pathophysiology, several anti-cytokines targeted against TNFα, IL-1β, IL-6 or IL-6 receptor and several cellular immunotherapies (anti-CD20 or CTLA-4Ig) have been successfully introduced for RA treatment. Studies have led to the recognition of TNFα as one of the cornerstone cytokines involved in synovial inflammatory process. Such results have provided the basis for the development of TNFα blocking agents (TBAs) for the treatment of RA. Five TNFα blocking agents (TBAs) are currently used for RA treatment, one corresponding to a recombinant soluble form of TNF receptor, TNFRSF1B (etanercept), four others corresponding to an anti-TNFα monoclonal antibody: infliximab, adalimumab (ADA), certolizumab and golimumab. These TBAs act by inhibiting the binding of TNFs to TNF receptors on cell surface and therefore interfering with TNF driven signal transduction pathways. Etanercept binds to both TNFα and TNFβ (also known as LymphoToxine A, LTA) while infliximab, adalimumab, certolizumab and golimumab bind to TNFα only.

The number of biological agents in RA is continuously increasing and various clinical trials with a TBA / methotrexate combination have shown efficacy in 60-70 % of RA patients. However, clinicians observe that around 30 to 40% of treated patients fail to respond to TBAs. Moreover, TNFα blocking agents may have side effects, they are costly and the efficacy of any given TBA in a given patient is unpredictable.

Taking into account the risk of these treatments, the increasing number of available therapeutic molecules in RA, the variability of the response to the various treatment, and to optimize the drug prescription, identification of predictive markers of TBA / methotrexate combination may be highly desirable.

### SUMMARY OF THE INVENTION:

The present invention relates to methods for determining whether a patient afflicted with rheumatoid arthritis will be a responder or a non-responder to a TNFα blocking agent treatment.

### DETAILED DESCRIPTION OF THE INVENTION:

The rheumatoid arthritis treatment response was investigated by the inventors using sera samples collected from RA patients receiving the methotrexate (MTX) / etanercept (ETA) treatment. The clinical efficacy was evaluated using the DAS28 score after 6 months of treatment according to the EULAR response criteria. Two cohorts of patients were investigated, the first is for identification of the combination of biomarkers, and the second one is for validation. Using the first cohort, the inventors performed a differential analysis between responder and non responder samples and revealed 12 differentially expressed serum biomarkers according to patient response. The inventors then used the combination of biomarkers to build a Random Forest statistical model to predict the patient's response. The inventors also validated the model by a blind test on a panel of patients. The inventors also validated the predictive biomarkers in peripheral blood mononuclear cells from the second cohort.

### Definitions:

The term "patient" denotes a mammal. In a preferred embodiment of the invention, a patient refers to any patient (preferably human) afflicted with rheumatoid arthritis. In another preferred embodiment of the invention, the term "patient" refers to any patient (preferably human) afflicted with rheumatoid arthritis receiving a methotrexate (MTX) first-line therapy.

The term "rheumatoid arthritis" refers to rheumatoid arthritis such as revised in the World Health Organisation Classification M05-M14.

The term "rheumatoid arthritis treatment" relates to methotrexate (MTX) and TNFα blocking agents (TBAs) combination treatment undergone by the rheumatoid arthritis patients. Typically, said treatment may be a methotrexate (MTX) and etanercept (ETA) combination.

The term "methotrexate" or "MTX" denotes the dihydrofolate reductase antagonist. Methotrexate inhibits cells proliferation by inhibiting purine metabolism and interfering with *de novo* DNA synthesis (Cronstein, 2005).

The term "TNFα" or "TNF-alpha" denotes the tumor necrosis factor - alpha. The human TNF-alpha is a human cytokine encoded by the TNF-alpha gene. TNF-alpha, a naturally occurring cytokine, plays a central role in the inflammatory response and in immune injury. It is formed by the cleavage of a precursor transmembrane protein, forming soluble molecules which aggregate to form trimolecular complexes. These complexes then bind to receptors found on a variety of cells. Binding produces an array of pro-inflammatory effects, including release of other pro-inflammatory cytokines, including IL-6, IL-8, and IL-1; release of matrix metalloproteinases; and up regulation of the expression of endothelial adhesion molecules, further amplifying the inflammatory and immune cascade by attracting leukocytes into extravascular tissues.

The term "TNFα blocking agent" or "TBA" refers to a molecule, such as protein, antibody, or small molecule that can significantly reduce TNFα properties. Such blocking agents include anti-TNFα antibodies, e.g. infliximab, adalimumab, CDP571 or D2E7, certolizumab and golimumab. Recombinant TNF-receptor based proteins have also been developed (e.g. etanercept, a recombinant fusion protein consisting of two soluble TNFα receptors joined by the Fc fragment of a human IgG1 molecule). A pegylated soluble TNF type 1 receptor can also be used as a TNF blocking agent. Additionally, thalidomide has been demonstrated to be a potent anti-TNF agent. TNFα blocking agents thus further include phosphodiesterase 4 (IV) inhibitor thalidomide analogues and other phosphodiesterase IV inhibitors.

The term "etanercept" or "ETA" denotes the tumor necrosis factor - alpha (TNFα) antagonist used for the treatment of rheumatoid arthritis. The term "etanercept" (ETA, ETN, Enbrel) is a recombinant TNF-receptor IgG-Fc-fusion protein composed of the p75 TNF receptor genetically fused to the Fc domain of IgG1. Etanercept neutralizes the proinflammatory cytokine tumor necrosis factor-α (TNFα) and lymphotoxin-α (Batycka-Baran et al., 2012).

The term "responder" refers to a rheumatoid arthritis patient that will respond to TNFα blocking agent treatment. The disease activity can be measured according to the standards recognized in the art. The "Disease Activity Score" (DAS) is a measure of the activity of rheumatoid arthritis. In Europe the DAS is the recognized standard in research and clinical practice. The following parameters are included in the calculation (Van Gestel AM, Prevoo MLL, van't Hof MA, et al. Development and validation of the European League Against Rheumatism response criteria for rheumatoid arthritis. Arthritis Rheum 1996; 39:34-40):
- Number of joints tender to the touch (TEN)
- Number of swollen joints (SW)
- Erythrocyte sedimentation rate (ESR) or C-reactive protein (CRP)
- Patient assessment of disease activity (VAS; mm)

The term "responder" refers to a rheumatoid arthritis patient that shows a decrease of DAS28 ≥1.2 after 3, 6 and 12 months of treatment. The term "responder" refers to a rheumatoid arthritis patient that shows DAS28 at the sixth month of < 3.2. The term "non-responder" refers to a rheumatoid arthritis patient that will not respond to TNFα blocking agent treatment. Good responders are defined as patients who have a decrease in DAS28 from baseline (ΔDAS28) of >1.2 and a DAS28 at the sixth month of < 3.2; moderate responders have either ΔDAS28 of >1.2 and a DAS28 at the sixth month of >3.2 or ΔDAS28 of 0.6 to 1.2 and a DAS28 at the sixth month of < 5.1; and non-responders are those who have either ΔDAS28 of < 0.6 or a DAS28 at the sixth month of >5.1. The term "non-responder" refers to a rheumatoid arthritis patient that shows a decrease of DAS28 of < 0.6. The term "non-responder" refers to a rheumatoid arthritis patient with a DAS28 score ≥ 3.2 (≥5.1) after 3, 6 and 12 months of treatment. A "responder" or "responsive" patient to a TNFα blocking agent treatment refers to a patient who shows or will show a clinically significant relief in the disease when treated with a TNFα blocking agent treatment.

The term "blood sample" refers to blood sample, a whole blood sample, a plasma sample, a serum sample or peripheral blood mononuclear cells (PBMCs).

All the biomarkers pertaining to the invention are known per se, and are listed in the below Table A.

| **Gene name** | **Polypeptide name** | **Polypeptide ID** |
|---|---|---|
| CO7 | Complement component C7 precursor | NP_000578 |
| PROS | Vitamin K-dependent protein S precursor | NP_000304 |
| S100A9 | Protein S100-A9 | NP_002956 |
| CERU | Ceruloplasmin precursor | NP_000087 |
| ITIH1 | Inter-alpha-trypsin inhibitor heavy chain H1 precursor | NP_002206 |
| ZA2G | Zinc-alpha-2-glycoprotein precursor | NP_001176 |
| PLNM | Plasminogen precursor | NP_000292 |
| ITIH3 | Inter-alpha-trypsin inhibitor heavy chain H3 precursor | NP_002208 |
| C1R | Complement C1r subcomponent precursor | NP_001724 |
| IC1 | Plasma protease C1 inhibitor precursor | NP_000053 |
| TRFE | Serotransferrin precursor | NP_001054 |
| CPN2 | Carboxypeptidase N subunit 2 precursor | NP_001073982 |

### Methods for predicting response

The present invention relates to a method of determining whether a patient afflicted with rheumatoid arthritis will be a responder or a non-responder to a TNFα blocking agent treatment comprising the steps of:
(i) measuring the expression level of at least one biomarker selected from the group consisting of CO7, PROS, S100A9, CERU, ITIH1, ZA2G, PLNM, ITIH3, C1R, IC1, TRFE and CPN2 in a blood sample obtained from said patient before the treatment,
(ii) comparing the expression level measured at step i) with a reference value,
(iii) detecting differential in the biomarker expression level between the blood sample and the reference value is indicative that said patient will be a responder or a non-responder.

In one embodiment, the TNFα blocking agent is etanercept (ETA).

A reference value is determined for each biomarker. Typically, the reference value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. Preferably, the person skilled in the art may compare the biomarkers expression levels (obtained according to the method of the invention with a defined threshold value). In one embodiment of the present invention, the threshold value is derived from the biomarkers expression level (or ratio, or score) determined in a blood sample derived from one or more patients who are responders to TNFα blocking agent treatment. In one embodiment of the present invention, the threshold value may also be derived from biomarker expression level (or ratio, or score) determined in a blood sample derived from one or more patients who are non-responders to TNFα blocking agent treatment. Furthermore, retrospective measurement of the biomarker expression levels (or ratio, or scores) in properly banked historical patient samples may be used in establishing these threshold values.

In a particular embodiment, the reference value may be determined by carrying out a method comprising the steps of
a) providing a collection of blood samples obtained from patients before the TNFα blocking agent
b) providing, for each blood sample provided at step a), information relating to the actual clinical outcome (response or no response);
c) providing a serial of arbitrary quantification values;
d) determining the level of the biomarker for each blood sample contained in the collection provided at step a);
e) classifying said blood samples in two groups for one specific arbitrary quantification value provided at step c), respectively: (i) a first group comprising blood samples that exhibit a quantification value for level that is lower than the said arbitrary quantification value contained in the said serial of quantification values; (ii) a second group comprising blood samples that exhibit a quantification value for said level that is higher than the said arbitrary quantification value contained in the said serial of quantification values; whereby two groups of blood samples are obtained for the said specific quantification value, wherein the blood samples of each group are separately enumerated;
f) calculating the statistical significance between (i) the quantification value obtained at step e) and (ii) the actual clinical outcome of the patients (i.e. response or not response) from which blood samples contained in the first and second groups defined at step f) derive;
g) reiterating steps f) and g) until every arbitrary quantification value provided at step d) is tested;
h) setting the said reference value as consisting of the arbitrary quantification value for which the highest statistical significance (most significant) has been calculated at step g).

For example the level of the biomarker has been assessed for 100 blood samples of 100 patients. The 100 samples are ranked according to the level of the biomarker. Sample 1 has the highest level and sample 100 has the lowest level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding patients, the p value between both subsets was calculated. The reference value is then selected such as the discrimination based on the criterion of the minimum p value is the strongest. In other terms, the level of the biomarker corresponding to the boundary between both subsets for which the p value is minimum is considered as the reference value. It should be noted that the reference value is not necessarily the median value of levels of the biomarker.

The setting of a single "cut-off" value thus allows discrimination between responder or non responder. Practically, high statistical significance values (e.g. low P values) are generally obtained for a range of successive arbitrary quantification values, and not only for a single arbitrary quantification value. Thus, in one alternative embodiment of the invention, instead of using a definite reference value, a range of values is provided. Therefore, a minimal statistical significance value (minimal threshold of significance, e.g. maximal threshold P value) is arbitrarily set and a range of a plurality of arbitrary quantification values for which the statistical significance value calculated at step g) is higher (more significant, e.g. lower P value) are retained, so that a range of quantification values is provided. This range of quantification values includes a "cut-off" value as described above. For example, on a hypothetical scale of 1 to 10, if the ideal cut-off value (the value with the highest statistical significance) is 5, a suitable (exemplary) range may be from 4-6. Therefore, a patient may be assessed by comparing values obtained by measuring the level of the biomarker, where values greater than 5 reveal that the patient will be a responder (or alternatively a non responder) and values less than 5 reveal that the patient will be a non responder (or alternatively a responder). In another embodiment, a patient may be assessed by comparing values obtained by measuring the level of the biomarker and comparing the values on a scale, where values above the range of 4-6 indicate that the patient will be a responder (or alternatively a non responder) and values below the range of 4-6 indicate that the patient will be a non responder (or alternatively a non responder), with values falling within the range of 4-6 indicating an intermediate response.

Typically, when the expression level determined for CO7, PROS, S100A9, CERU, ITIH1, ZA2G, PLNM, ITIH3, C1R, IC1 or CPN2 is higher than the corresponding reference value, it is concluded that the patient will be a responder to TNFα blocking agent treatment, and accordingly, when the expression level determined for CO7, PROS, S100A9, CERU, ITIH1, ZA2G, PLNM, ITIH3, C1R, IC1 or CPN2 is lower than the corresponding reference value the patient will be a non-responder to TNFα blocking agent treatment.

Typically, when the expression level determined for TRFE is lower than the corresponding reference value it is concluded that the patient will be a responder to TNFα blocking agent treatment, and accordingly, when the expression level determined for TRFE is higher than the corresponding reference value it is concluded that the patient will be a non responder to TNFα blocking agent treatment.

In some embodiments, the expression levels of 2, 3, 4, 5, 6, 7, 8, 9, 10, 11, or 12 biomarkers are measured.

In one embodiment, the present invention comprises measuring the expression levels of PROS and CO7 are determined. Accordingly, the present invention relates to a method of determining whether a patient afflicted with rheumatoid arthritis will be a responder or a non-responder to a TNFα blocking agent treatment comprising the steps of (i) measuring the expression level of PROS and CO7 in a blood sample obtained from said patient before the treatment, (ii) comparing the expression levels measured at step i) with their corresponding reference values, and (iii) and concluding that the patient will be a responder when both expression levels measured at step i) are higher that their corresponding reference values.

In a particular embodiment, the expression levels of CO7, PROS, S100A9, CERU, ITIH1, ZA2G, PLNM, ITIH3, C1R, IC1, TRFE and CPN2 are measured.

In another particular embodiment, a score which is a composite of the expression levels of the different biomarkers may also be determined and compared to a reference value wherein a difference between said score and said reference value is indicative whether said patient is a responder or a non-responder to TNFα blocking agent treatment.

In a particular embodiment, the score may be generated by a computer program.

### Methods for determining the expression level of a biomarker

Methods for measuring the expression level of a biomarker in a blood sample may be assessed by any of a wide variety of well-known methods from one of skill in the art for detecting expression of a protein including, but not limited to, direct methods like mass spectrometry-based quantification methods, protein microarray methods, enzyme immunoassay (EIA), radioimmunoassay (RIA), Western blot analysis, ELISA, Luminex, ELISPOT and enzyme linked immunoabsorbant assay and undirect methods based on detecting expression of corresponding messenger ribonucleic acids (mRNAs) and or corresponding micro RNAs (miRNAs). The mRNA and or miRNA expression profile may be determined by any technology known by a man skilled in the art. In particular, each mRNA and/or miRNA expression level may be measured using any technology known by a man skilled in the art, including nucleic microarrays, quantitative Polymerase Chain Reaction (qPCR), next generation sequencing and hybridization with a labelled probe.

Said direct analysis can be assessed by contacting the blood sample with a binding partner capable of selectively interacting with the biomarker present in the blood sample. The binding partner may be an antibody that may be polyclonal or monoclonal, preferably monoclonal (e.g., a isotope-label, element-label, radio-labeled, chromophore- labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, etc.) which binds specifically to the protein translated from the gene encoding for CO7, PROS, S100A9, CERU, ITIH1, ZA2G, PLNM, ITIH3, C1R, IC1, TRFE and CPN2. In another embodiment, the binding partner may be an aptamer.

Polyclonal antibodies of the invention or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.

Monoclonal antibodies of the invention or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985).

Alternatively, techniques described for the production of single chain antibodies (see e.g. U.S. Pat. No. 4,946,778) can be adapted to produce anti-biomarker, single chain antibodies. Antibodies useful in practicing the present invention also include anti-biomarker fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to biomarker. For example, phage display of antibodies may be used. In such a method, single-chain Fv (scFv) or Fab fragments are expressed on the surface of a suitable bacteriophage, e. g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay.

In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al., 1996).

The binding partners of the invention such as antibodies or aptamers, may be labelled with a detectable molecule or substance, such as an isotope, an element, a fluorescent molecule, a radioactive molecule or any others labels known in the art. Labels are known in the art that generally provide (either directly or indirectly) a signal.

As used herein, the term "labelled", with regard to the antibody, is intended to encompass direct labelling of the antibody or aptamer by coupling (i.e., physically linking) a detectable substance, such as an isotope, an element, a radioactive agent or a fluorophore (e.g. fluorescein isothiocyanate (FITC) or phycoerythrin (PE) or Indocyanine (Cy5)) to the antibody or aptamer, as well as indirect labelling of the probe or antibody by reactivity with a detectable substance. An antibody or aptamer of the invention may be produced with a specific isotope or a radioactive molecule by any method known in the art. For example radioactive molecules include but are not limited to radioactive atom for scintigraphic studies such as I123, I124, In111, Re186, Re188, specific isotopes include but are not limited to 13C, 15N, 126I, 79Br, 81Br.

The afore mentioned assays generally involve the binding of the binding partner (ie. antibody or aptamer) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidene fluoride; diazotized paper; nylon membranes; activated beads, magnetically responsive beads, silicon wafers.

In a particular embodiment, an ELISA method can be used, wherein the wells of a microtiter plate are coated with a set of antibodies which recognize said biomarker(s). A blood sample containing or suspected of containing said biomarker(s) is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art such as Singulex, Quanterix, MSD, Bioscale, Cytof.

In one embodiment, an Enzyme-linked immunospot (ELISpot) method may be used. Typically, the blood sample is transferred to a plate which has been coated with the desired anti- biomarker capture antibodies. Revelation is carried out with biotinylated secondary Abs and standard colorimetric or fluorimetric detection methods such as streptavidin-alkaline phosphatase and NBT-BCIP and the spots counted.

In one embodiment, when multi- biomarker expression measurement is required, use of beads bearing binding partners of interest may be preferred. In a particular embodiment, the bead may be a cytometric bead for use in flow cytometry. Such beads may for example correspond to BD™ Cytometric Beads commercialized by BD Biosciences (San Jose, California). Typically cytometric beads may be suitable for preparing a multiplexed bead assay. A multiplexed bead assay, such as, for example, the BD^{(™)} Cytometric Bead Array, is a series of spectrally discrete beads that can be used to capture and quantify soluble antigens. Typically, beads are labelled with one or more spectrally distinct fluorescent dyes, and detection is carried out using a multiplicity of photodetectors, one for each distinct dye to be detected. A number of methods of making and using sets of distinguishable beads have been described in the literature. These include beads distinguishable by size, wherein each size bead is coated with a different target-specific antibody (see e.g. Fulwyler and McHugh, 1990, Methods in Cell Biology 33:613-629), beads with two or more fluorescent dyes at varying concentrations, wherein the beads are identified by the levels of fluorescence dyes (see e.g. European Patent No. 0 126,450), and beads distinguishably labelled with two different dyes, wherein the beads are identified by separately measuring the fluorescence intensity of each of the dyes (see e.g. U.S. patent Nos. 4,499,052 and 4,717,655). Both one-dimensional and two-dimensional arrays for the simultaneous analysis of multiple antigens by flow cytometry are available commercially. Examples of one-dimensional arrays of singly dyed beads distinguishable by the level of fluorescence intensity include the BD^{(™)} Cytometric Bead Array (CBA) (BD Biosciences, San Jose, Calif.) and Cyto-Plex^{(™)} Flow Cytometry microspheres (Duke Scientific, Palo Alto, Calif.). An example of a two-dimensional array of beads distinguishable by a combination of fluorescence intensity (five levels) and size (two sizes) is the QuantumPlex^{(™)} microspheres (Bangs Laboratories, Fisher, Ind.). An example of a two-dimensional array of doubly-dyed beads distinguishable by the levels of fluorescence of each of the two dyes is described in Fulton et al. (1997, Clinical Chemistry 43(9):1749-1756). The beads may be labelled with any fluorescent compound known in the art such as e.g. FITC (FL1), PE (FL2), fluorophores for use in the blue laser (e.g. PerCP, PE-Cy7, PE-Cy5, FL3 and APC or Cy5, FL4), fluorophores for use in the red, violet or UV laser (e.g. Pacific blue, pacific orange). In another particular embodiment, bead is a magnetic bead for use in magnetic separation. Magnetic beads are known to those of skill in the art. Typically, the magnetic bead is preferably made of a magnetic material selected from the group consisting of metals (e.g. ferrum, cobalt and nickel), an alloy thereof and an oxide thereof. In another particular embodiment, bead is bead that is dyed and magnetized.

In one embodiment, protein microarray methods may be used. Typically, at least one antibody or aptamer directed against the biomarker(s) is immobilized or grafted to an array(s), a solid or semi-solid surface(s). A blood sample containing or suspected of containing the biomarker(s) is then labelled with at least one isotope or one element or one fluorophore or one colorimetric tag that are not naturally contained in the tested blood sample. After a period of incubation of said blood sample with the array sufficient to allow the formation of antibody-antigen complexes, the array is then washed and dried. After all, quantifying said biomarkers may be achieved using any appropriate microarray scanner like fluorescence scanner, colorimetric scanner, SIMS (secondary ions mass spectrometry) scanner, maldi scanner, electromagnetic scanner or any technique allowing to quantify said labels.

In another embodiment, the antibody or aptamer grafted on the array is labelled.

In another embodiment, reverse phase arrays may be used. Typically, at least one blood sample is immobilized or grafted to an array(s), a solid or semi-solid surface(s). An antibody or aptamer against the suspected biomarker(s) is then labelled with at least one isotope or one element or one fluorophore or one colorimetric tag that are not naturally contained in the tested blood sample. After a period of incubation of said antibody or aptamer with the array sufficient to allow the formation of antibody-antigen complexes, the array is then washed and dried. After all, detecting quantifying and counting by D-SIMS said biomarkers containing said isotope or group of isotopes, and a reference natural element, and then calculating the isotopic ratio between the biomarkers and the reference natural element. may be achieve using any appropriate microarray scanner like fluorescence scanner, colorimetric scanner, SIMS (secondary ions mass spectrometry) scanner, maldi scanner, electromagnetic scanner or any technique allowing to quantify said labels.

In one embodiment, said direct analysis can also be assessed by mass Spectrometry. Mass spectrometry-based quantification methods may be performed using either labelled or unlabelled approaches (DeSouza and Siu, 2012). Mass spectrometry-based quantification methods may be performed using chemical labeling, metabolic labelingor proteolytic labeling. Mass spectrometry-based quantification methods may be performed using mass spectrometry label free quantification, LTQ Orbitrap Velos, LTQ-MS/MS, a quantification based on extracted ion chromatogram EIC (progenesis LC-MS, Liquid chromatography-mass spectrometry) and then profile alignement to determine differential expression of biomarkers.

In one embodiment, undirect analysis may be used. Said undirect analysis can be assessed by measuring:
Either mRNAs corresponding to biomarkers from a whole blood sample. It also includes specific cellular subtypes or derivatives extracted from those such as PBMCs.
And/or either miRNA corresponding to biomarkers from a serum sample, a plasma sample, a blood sample, in particular a peripheral blood sample, a lymph sample (see Weber et al.,2010). It also includes specific cellular subtypes or derivatives extracted from those such as PBMCs.

In a preferred embodiment, the mRNA and or miRNA expression profile is determined using quantitative PCR. Quantitative, or real-time, PCR is a well-known and easily available technology for those skilled in the art.

In another preferred embodiment, the miRNA expression profile is determined by the use of a nucleic microarray. A "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or RNAs, or DNA or RNA oligonucleotides ("oligonucleotide microarray"), and the oligonucleotides may be about 25 to about 60 base pairs or less in length. To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art.

Peripheral blood samples can be collected in PAXgene tubes (PreAnalytiX). PAXgene blood miRNA kit (Qiagen) can be used to extract total RNA (mRNA, miRNAs, rRNA and tRNA) from blood.

### Kits of the invention

The invention also relates to a kit for performing the methods as above described, wherein said kit comprises means for measuring the expression level of at least one biomarker selected from the group consisting of CO7, PROS, S100A9, CERU, ITIH1, ZA2G, PLNM, ITIH3, C1R, IC1, TRFE and CPN2 that are indicative of patient responder to rheumatoid arthritis treatment. In particular embodiment the kit comprises means for measuring the expression levels of CO7 and PROS. Typically the kit may include an antibody, or a set of antibodies as above described. In a particular embodiment, the antibody or set of antibodies are labelled as above described. In particular embodiment the kit may comprise at least one antibody directed to CO7 and at least one antibody directed to PROS. The kit may also contain other suitably packaged reagents and materials needed for the particular detection protocol, including solid-phase matrices, if applicable, and standards.

### Methods of treatment

The method of the invention allows to define a subgroup of patients who will be responder or non responder to the TNFα blocking agent treatment.

A further object of the invention relates to a method for the treatment of rheumatoid arthritis in a patient in need thereof comprising the steps of:
a) determining whether a patient afflicted with rheumatoid arthritis will be a responder or a non-responder to a TNFα blocking agent treatment by performing the method according to the invention,
b) administering the TNFα blocking agent treatment, if said patient has been considered as a responder.

A further object of the invention relates to a methotrexate (MTX) and TNFα blocking agent for use in the treatment of rheumatoid arthritis in a patient in need thereof, wherein the patient was being classified as responder by the method as above described.

A further object of the invention relates to a methotrexate (MTX) and etanercept (ETA) for use in the treatment of rheumatoid arthritis in a patient in need thereof, wherein the patient was being classified as responder by the method as above described.

The invention will be further illustrated by the following figures and examples. However, these examples and figures should not be interpreted in any way as limiting the scope of the present invention.

### FIGURES:

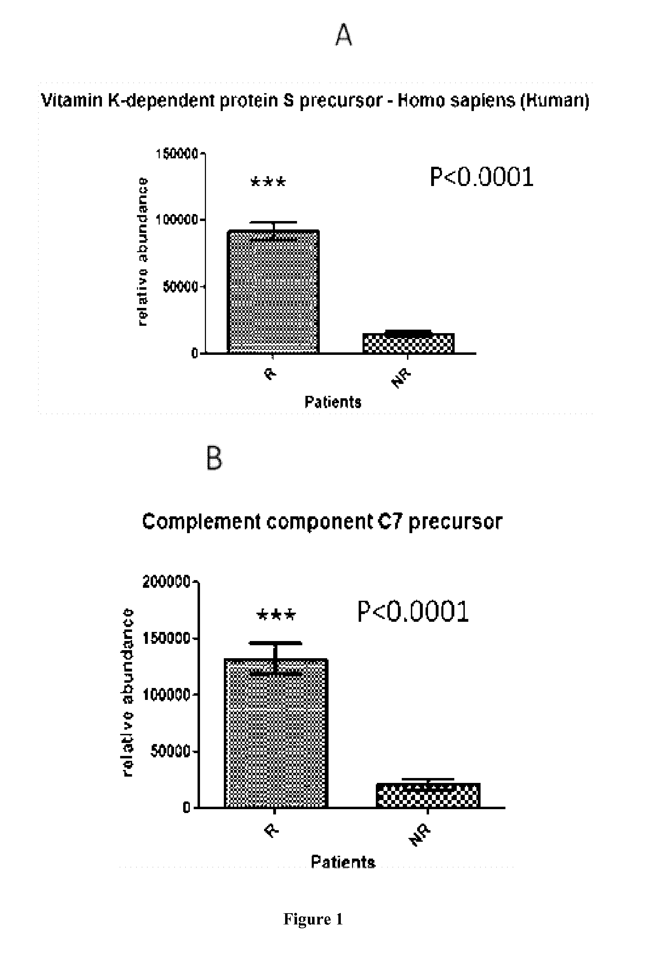

**Figure 1****: Extracellular overexpression of C07 and PROS proteins in responders.** Relative quantification of serum protein PROS (A) and CO7 (B) at baseline in responders versus non-responders to an etanercept/ methotrexate combination by mass spectrometry "label free" approach. Significant difference is noted by asterisk (*p* < *0,05, Mann and* Whitney's test). The results are presented as mean+SEM (A and B).

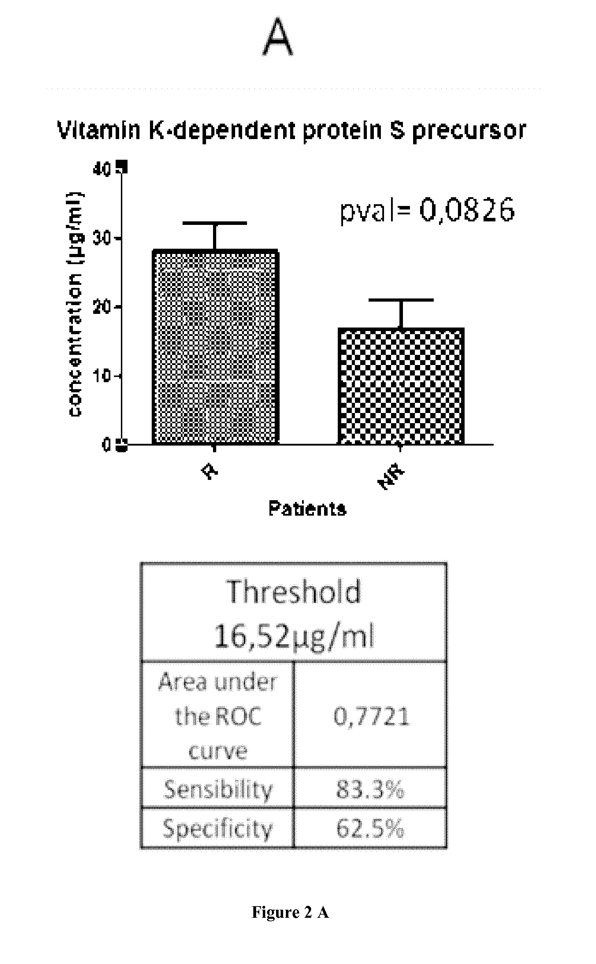

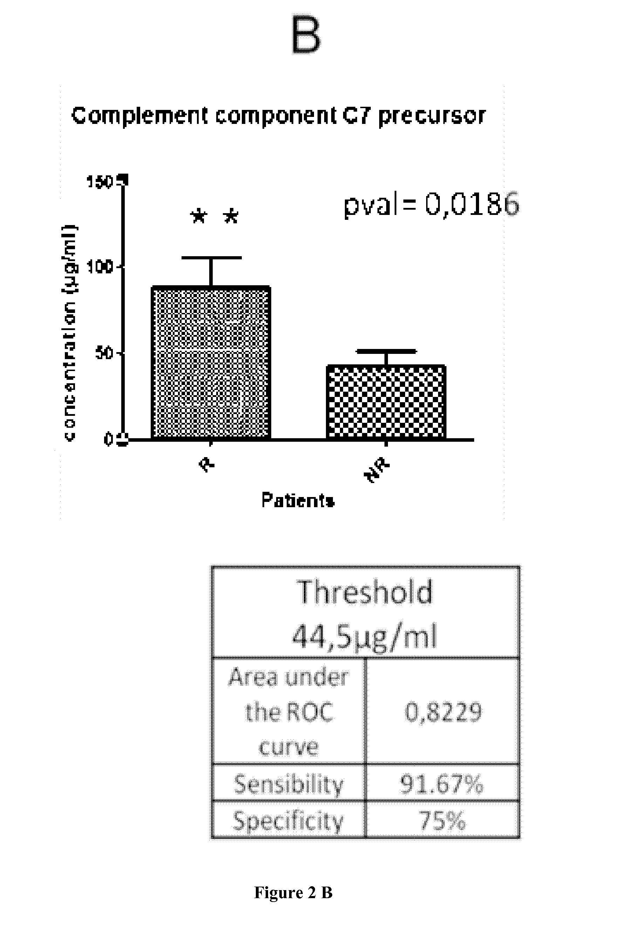

**Figure 2****: Extracellular overexpression of PROS (B) and CO7(C) proteins in responders.** Absolute quantification of serum PROS (B) and CO7(C) proteins at baseline in responders versus non-responders by ELISA. Significant difference is noted by asterisk (*p* < *0,05,* Mann- Whitney's test). The results are presented as mean+SEM (A and B). Threshold was determined by ROC method.

### EXAMPLE:

### Material & Methods

### Patients

A first cohort of 22 patients (cohort "discovery") and a second cohort of 20 patients (cohort "validation") with active RA were treated by a subcutaneous injection of ETA (50 mg / week) in combination with MTX. The clinical efficacy of this combination was evaluated after six months of therapy according to the EULAR response criteria. The patients were categorized into good, moderate or non-responders based on the amount of change in the DAS28 and the level of DAS28 reached. Good responders are defined as patients who have a decrease in DAS28 from baseline (ΔDAS28) of >1.2 and a DAS28 at the sixth month of < 3.2; moderate responders have either ΔDAS28 of >1.2 and a DAS28 at the sixth month of >3.2 or ΔDAS28 of 0.6 to 1.2 and a DAS28 at the sixth month of < 5.1; and non-responders are those who have either ΔDAS28 of < 0.6 or a DAS28 at the sixth month of >5.1. For proteomic analysis, a serum sample was carried out in patients prior to treatment exposure.

### Serum samples

The blood samples were allowed to coagulate and then centrifuged at 2800 rpm/min for 10 min. The serum samples were stored at -80°C until analysed. The protein concentration of this serum samples was determined using the Bradford method.

### Peptides extraction

Twenty-five µg of samples were loaded on polyacrylamide gels and allowed for a short period a migration (1H30) in a stacking gel (7%). After staining, the revelated protein band was excised and the proteins within the bands were reduced in 5mM dithithreitol and cysteins irreversibly alkylated in 25 mM iodoacetamide. After washing steps with water, gel bands were submitted to protein digestion (trypsin from Promega, 0,5µg per band). Several steps of peptide extraction were then performed in H20/CH3CN/TFA mixtures (50/50/1) and the peptide fractions were combined and evaporated with a SpeedVac (Savant)

### Liquid nanochromatography and mass spectrometry

For mass spectrometric analyses, peptides were dissolved in 0.1% formic acid in water. All experiments were carried out with a linear quadrupole ion trap-Orbitrap mass spectrometer (LTQ Orbitrap Velos, Thermo Scientific, Germany) equipped with a nano-ESI source and coupled to a nanoliquid chromatrography (Easy-nLC II, Thermo Scientific). The sample was loaded onto the enrichment column (Cap Trap C8, 0.5 x 2 mm, Michrom Bioresources) at a pressure of 200 bar in 0.1% FA. The separation was done with a reversed phase column (C18, L153, ID 5µm, 100 Å pore size, Nikkyo Technos, Japan). The liquid chromatography gradient (mobile phase A: H2O/0.1% FA; mobile phase B: ACN/0.1% FA) was delivered at a flow rate of 300 nl/min. Tryptic peptides were eluted from the reverse-phase column into the mass spectrometer, using a linear gradient of 15% to 55% B over 30 min. The capillary voltage was set at 1.5 kV; the source temperature was 200 °C. The mass spectrometer was operated in the data-dependent mode to automatically switch between Orbitrap-MS and LTQ-MS/MS acquisition. Survey full scan mass spectra (from m/z 300 to 2000) were acquired in the Orbitrap with a resolution of R = 30,000. The mass spectrometer selected the 20 most intense ions. Target peptides already selected for MS/MS were dynamically excluded for 30 s. General mass spectrometry conditions for selection were: ion selection threshold was 500 counts for MS/MS, and an activation Q-value of 0.25 and activation time of 10 ms were applied for MS/MS. Label-free peptide quantification RAW data were imported in Progenesis LC-MS software (v4.0.4441.29989). Profile data of the MS scans were transformed to peak lists with Progenesis LC-MS using a proprietary algorithm. One sample was set as a reference, and the retention times of all other samples within the experiment were aligned (15 manual landmarks, followed by automatic alignment). Features with only one charge or more than 8 charges were excluded from further analyses. After alignment and feature exclusion, raw abundances of all features were normalized. Normalization results in a unique factor for each sample that corrects all features in the sample. Samples were divided into the responders group and non-responders groups, and statistical analysis was performed using normalized abundances for one-way analysis of variance (ANOVA) calculations of all remained features. No minimal thresholds were set for the selection of data to use for quantification. But only features presented q-value >0,05 and P-value>0,05 were selectionned to realize a principal component analysis (ACP). MS/MS spectra from peptides selectionned were exported from the Progenesis LC-MS software as Mascot Generic file (mgf) and used for peptide identification with Mascot (version 2.2) in the SwissProt database for human (*Homo Sapiens;* Sprot 55.6;390696 sequences; 140503634 residues). Following search parameters were used: 10 ppm peptide mass tolerance and 0.5 Da fragment mass tolerance, one missed cleavage was allowed, carbamidomethylation (C) was set as fixed modification and oxidation (M), deamidation was as variable modifications. Only peptides with ion scores of 15 were considered and re-imported into Progenesis. After identification, peptides of an identified protein were included and the total cumulative abundance was calculated by summing the abundances of all peptides allocated to the respective protein.

### Determination of serum levels of CO7 and PROS by ELISA

Serum levels of CO7 and PROS were determined in 22 RA patients at baseline in sera from patient responders and non-responders, using enzyme-linked immunosorbent assay (ELISA) according to the manufacturer's instructions (USCNK, USA .EIAAB,China).

### Statistical analyses

The Kolmogorov-Smirnov test was used to evaluate the data distributions. Accordingly, Mann Whitney non-parametric tests were used to compare for comparison of medians proteins levels from label free experiments and from ELISA. Mann Whitney non-parametric tests were also used to compare difference of clinical and demographic data between all responders versus non-responders at baseline.

The R software was used for Unsupervised hierarchical clustering analysis, using Pearson and WARD linkage options, separated the responders and non-responders to the MTX/ETA combination.

### Results

### RA patients and response to treatment

Table 1 shows demographic, clinical and biological informations for "discovery" cohort with 22 patients at study initiation. Twelve patients were classified as responders and ten as non-responders to an etanercept/ methotrexate combination, at six months according to the EULAR criteria. By definition, the DAS28 score improved at 6 months in responders population (delta DAS28 =-2.57 ± 0.18), whereas it was getting worse in non-responders population (DAS28= 0.17 ± 0.17). Before treatment, four parameters (CRP, DAS, ESR, HAQ) were slightly higher in responders compared to non-responders but the difference was not significant (all P-value>0.05). Only two parameters were higher in non-responders population (morning siftness and pain) and the difference between these groups was again not significant. Finally, the sex ratio was different in this population but women were far more represented in both R and NR populations.

Thus, samples were collected in two similar populations, and the only variable between the two populations will be the response to treatment at 6 months. Table 2 shows demographic, clinical and biological informations for "validation" cohort with 20 patients at study initiation. Ten patients were classified as responders and eight as non-responders to an etanercept/ methotrexate combination, at six months according to the EULAR criteria. These parameters showed no significant difference at study initiation between the 2 groups. With "discovery" and "validation" cohorts, we have similar populations to achieve differential analysis of serum proteomes of patients before the start of treatment.

**Table 1: Demographic, clinical and biological data of RA patients at baseline:**

| | **Population 1 (serum)** | | |
|---|---|---|---|
| | **Responders (n=12)** | **Non Responders (n=10)** | **p-value** |
| | | | |
| Age (years) | 51.08 ± 3.83 | 58.74 ± 4.37 | 0.12 |
| Sex (f/m) | 08/04 | 09/01 | - |
| Methotrexate | 15.0± 1.36 | 13.30 ± 1.87 | 0.51 |
| (mg/week) | | | |
| Morning stiffness | 47.27 ± 16.48 | 61.88 ± 27.12 | 0.62 |
| (minutes) | | | |
| Pain | 61.82 ± 4.78 | 63.75 ± 6.03 | 0.77 |
| (0-100 mm VAS) | | | |
| ESR | 27.27 ± 6.22 | 22.00 ± 5.20 | 0.51 |
| (mm/hour) | | | |
| CRP | 20.59 ± 7.9 | 10.07 ± 5.75 | 0.14 |
| (mg/l) | | | |
| HAQ score | 1.38 ± 0.31 | 1.09 ± 0.16 | 0.60 |
| (0-3) | | | |
| DAS28 | 4.17± 0.26 | 3.41 ± 0.34 | 0.12 |
| DAS28 | 1.61 ± 0.16 | 3.59 ± 0.32 | 0.0004 |
| 6 month | | | |
| Δ DAS28 | -2.57 ± 0.18 | 0.17 ± 0.17 | < 0.0001 |

Values are mean ± SEM. All differences between responders versus non-responders at baseline of the first cohort were non-significant (p-values >0.05. Mann Whitney non-parametric test). Only DAS28 at 6 month and Delta DAS28 showed a significant difference between R and NR patients. CRP. C-reactive protein; DAS. disease activity score at initiation of treatment ; Δ DAS28. Difference between 6 months and baseline; ESR. erythrocyte sedimentation rate; HAQ. Health Assessment Questionnaire; VAS. visual analogue scale (patient's assessment of pain).

**Table 2: Demographic, clinical and biological data of RA patients at baseline:**

| | **Population 2 (serum)** | | |
|---|---|---|---|
| | **Responders (n=12)** | **Non Responders (n=8)** | **p-value** |
| | | | |
| Age (years) | 47.57 ± 4.79 | 56.51 ± 3.09 | 0.18 |
| Sex (f/m) | 9/3 | 6/2 | - |
| Methotrexate | 12.73± 2.06 | 8.75 ± 2.22 | 0.10 |
| (mg/week) | | | |
| Morning stiffness | 112.3± 44.59 | 72.50 ± 35.49 | 0.28 |
| (minutes) | | | |
| Pain | 56.36 ± 7.2 | 52.63 ±7.89 | 0.71 |
| (0-100 mm VAS) | | | |
| ESR | 25.55 ± 7.46 | 38.13 ± 10.12 | 0.53 |
| (mm/hour) | | | |
| CRP | 45.31 ±12.18 | 33.23 ± 14.15 | 0.54 |
| (mg/l) | | | |
| DAS28 | 4.24± 0.26 | 4.22 ± 0.26 | 0.61 |
| DAS28 | 1.89 ± 0.18 | 3.64 ± 0.29 | 0.001 |
| 6 month | | | |
| Δ DAS28 | -2.27 ± 0.34 | -0.57 ± 0.4 | 0.012 |

Values are mean ± SEM. All differences between responders versus non-responders at baseline of the second cohort were non-significant (p-values >0.05. Mann Whitney non-parametric test). In the second cohort, only DAS28 at 6 month and Delta DAS28 showed a significant difference between R and NR patients. CRP. C-reactive protein; DAS. disease activity score at initiation of treatment ; Δ DAS28. Difference between 6 months and baseline; ESR. erythrocyte sedimentation rate; VAS. visual analogue scale (patient's assessment of pain).

### Identification of 12 biomakers able to separate good and non-responders

From the "discovery" cohort, the inventors performed a differential analysis of the serum proteome samples responders and non responders before treatment. This ProGenesis quantification performed with LC-MS occured in two stages. First, a selection of peptides showed a differential expression between the two groups (P-value <0.05 and Q-value <0.05), followed by an identification of these peptides. 267 peptides presented differential expression between the two groups. Only proteotryptic peptides were used for quantification and the resulting proteins were identified by at least two peptides. 12 proteins had a significant differential expression between the two groups, including Complement component C7 (CO7) and Vitamin K-dependent protein S precursor (PROS) that are significantly overexpressed (pvalue <0.001) in responding patients before the start of treatment (Figure 1). The relative abundance values obtained for each protein combination helped achieving unsupervised classification between patients. This combination allowed to split into two distinct groups (responders and non-responders) with a specificity of 100% and a sensitivity of 91.67%.

### Validation of two proteins by absolute quantification

With samples of the cohort "validation", we have studied the expression of two proteins in combination, referred as to PROS and CO7. The inventors carried out ELISA assays with antibodies against proteins of interest (Figure 2). The CO7 protein is significantly overexpressed in responders (P-value: 0.0186), while the protein PROS is not significantly overexpressed in responding patients (P-value: 0.0826). Absolute quantification of these proteins revealed for each protein a concentration threshold associated to a right answer. The threshold of PROS is 16.52 mg/ml and can discriminate good responders and non-responders with a sensitivity of 83.3% and a specificity of 62.5. The threshold of CO7 is 44.5 mcg/ml and can discriminate good responders and non-responders with a sensitivity of 91.67% and a specificity of 75%. Using the combination of the two concentration thresholds, the sensitivity and specificity are 75% and 100% respectively. None of the non-responders are simultaneously present above the threshold concentration of the two proteins. This combination of two proteins identifies non responders cohort "validation".

### REFERENCES:

Throughout this application, various references describe the state of the art to which this invention pertains. The disclosures of these references are hereby incorporated by reference into the present disclosure.

## Claims

1. A method of determining whether a patient afflicted with rheumatoid arthritis will be a responder or a non-responder to a TNFα blocking agent treatment comprising the steps of:
(i) measuring the expression level of at least one biomarker selected from the group consisting of CO7, PROS, S100A9, CERU, ITIH1, ZA2G, PLNM, ITIH3, C1R, IC1, TRFE and CPN2 in a blood sample obtained from said patient before the treatment,
(ii) comparing the expression level measured at step i) with a reference value,
(iii) detecting differential in the biomarker expression level between the blood sample and the reference value is indicative that said patient will be a responder or a non-responder.

2. The method according to claim 1 wherein said TNFα blocking agent is etanercept.

3. A kit for performing the method according to any one of claims 1 or 2 wherein said kit comprises means for measuring the expression level of at least one biomarker selected from the group consisting of CO7, PROS, S100A9, CERU, ITIH1, ZA2G, PLNM, ITIH3, C1R, IC1, TRFE and CPN2 that are indicative of patient responder to rheumatoid arthritis treatment.
